# DEMANDE DE BREVET EUROPEEN

(11) **EP 4 124 349 A1**
(43) Date de publication de la demande: **01.02.2023**
(21) Numéro de dépôt: 21188060.4
(22) Date de dépôt: 27.07.2021
(51) Int. Cl.: A61L 2/07, A61L 2/26, B01D 1/00, B01B 1/00

(54) **ACCESSOIRE POUR PURIFICATEUR-VAPEUR**

(71) Demandeur: Laurastar SA, 1618 Châtel-St-Denis (CH)
(72) Inventeur: Denisart, Jean-Luc, 1096 Cully (CH)
(74) Mandataire: Grosfillier, Philippe

(57) **Abrégé**

Accessoire (1,14) pour purificateur-vapeur (12) comprenant un support (2, 15) d'objet (4-6) à purifier, au moins une sortie de vapeur (3) disposée de manière à assurer une vaporisation de l'extérieur et/ou de l'intérieur dudit objet (4-6), une entrée de vapeur (7) communiquant avec ladite sortie (3) ; caractérisé par le fait qu'il comprend également un connecteur fluidique (8) permettant de relier ladite entrée (7) à une buse de purificateur-vapeur (9) et de fixer de manière amovible l'accessoire (1,14) à ladite buse (9).

## Description

### Domaine de l'invention

L'invention concerne la purification d'objets au moyen de vapeur, et plus précisément les dispositifs utilisés à cet effet.

### Etat de la technique

La purification d'objets, p.ex. la stérilisation ou la décontamination, au moyen de vapeur est décrite dans de nombreux documents de l'état de la technique, en particulier dans les demandes de brevet EP 0 016 889 A1 et EP 0 155 933 A2. Plus récemment, des purificateurs-vapeur portables ont été proposés, en particulier le modèle IGGI^{™} de la demanderesse. Avec ce type de dispositif, les objets à purifier sont tenus à la main ou posés sur une surface horizontale (p.ex. une table) ou attachés à un support (p.ex. crochet ou cintre).

Bien que cette méthode soit aisément applicable au traitement de nombreux objets, notamment les textiles et des objets de dimensions relativement importantes, elle n'est pas aussi efficace lorsqu'il s'agit d'objets de dimensions relativement faibles (clés, lunettes, ustensiles, etc...) ou d'objets creux (bouteilles, gourdes, biberons, bocaux, etc...). En outre, si ce type d'objet est tenu à la main, le risque de se brûler est plus élevé.

Il serait donc souhaitable de purifier efficacement et sans danger ce type d'objet avec un purificateur-vapeur portable.

### Description générale de l'invention

Les problèmes décrits dans le chapitre précédent peuvent être évités grâce à la présente invention. Celle-ci a pour objet un accessoire pour purificateur-vapeur comprenant un support d'objet à purifier, au moins une sortie de vapeur disposée de manière à assurer une vaporisation de l'extérieur et/ou de l'intérieur dudit objet, une entrée de vapeur communiquant avec ladite sortie. L'accessoire selon l'invention se caractérise par le fait qu'il comprend également un connecteur fluidique permettant de relier ladite entrée à une buse de purificateur-vapeur et de fixer de manière amovible l'accessoire à ladite buse.

L'objet à purifier est donc déposé sur un support qui est rendu solidaire du purificateur-vapeur, au niveau de sa buse de sortie. L'ensemble ainsi formé constitue un dispositif unique qui nécessite l'usage que d'une main.

Le fait que l'accessoire soit amovible permet d'utiliser différents modèles de support, choisis en fonction de l'objet à purifier. De la sorte, il est également possible d'utiliser le purificateur-vapeur de manière classique, à savoir sans accessoire.

De préférence, le transfert de la vapeur entre ladite entrée et ladite sortie est réalisé à travers le support.

L'accessoire peut également contenir plusieurs entrées et/ou plusieurs sorties de vapeur.

Avantageusement, l'accessoire est de forme cylindrique et le connecteur orienté perpendiculairement par rapport à l'axe de symétrie de l'accessoire.

Selon une variante de l'invention, particulièrement avantageuse pour le traitement d'objets creux, l'accessoire comprend un réservoir de condensat, disposé de préférence dans la partie inférieure de l'accessoire. Le liquide récupéré est ainsi déplacé sous l'effet de la force de gravitation.

Selon un mode de réalisation particulièrement adapté au traitement d'objets creux, l'accessoire comprend un tube creux comportant plusieurs sorties de vapeur, principalement disposées sur sa face latérale, et dont l'une des extrémités est fixée au support, de préférence au centre de celui-ci. Les dimensions du tube creux sont déterminées de façon à ce qu'il soit disposé dans la cavité de l'objet creux à traiter.

L'accessoire peut également comprendre un élément d'extension de support monté de manière amovible sur le support. De la sorte, des objets de dimensions plus importantes peuvent être utilisés.

Si le support a la forme d'un disque, l'élément d'extension peut se présenter sous la forme d'un anneau qui se fixe et s'étend le long de la périphérie du disque.

Selon un autre mode de réalisation de l'invention, particulièrement destiné à des objets de dimensions relativement faibles et/ou de géométries diverses, le support se présente sous la forme d'un récipient.

Avantageusement, le récipient comprend un couvercle qui de préférence, assure une étanchéité optimale avec l'extérieur du récipient.

De préférence, le couvercle comporte au moins un orifice qui assure l'évacuation de la vapeur vers l'extérieur, pour éviter une surpression à l'intérieur du récipient mais également un écoulement optimal de la vapeur le long de l'objet à traiter.

### Description détaillée de l'invention

L'invention est décrite de manière plus détaillée dans ce chapitre, notamment au moyen d'exemples et de figures qui sont brièvement résumées comme suit :
Figure 1 : Accessoire type I
Figure 2 : Accessoire de la figure 1 avec élément d'extension de support
Figure 3 : Élément d'extension de support. Vue dessus (3a) et vue dessous (3b)
Figure 4 : Accessoire de la figure 1 fixé à un purificateur-vapeur
Figure 5 : Accessoire de la figure 2 fixé à un purificateur-vapeur
Figure 6 : Ensemble de la figure 4 avec objet tubulaire
Figure 7 : Ensemble de la figure 5 avec objet cylindrique
Figure 8 : Écoulement de la vapeur dans l'ensemble de la figure 7
Figure 9 : Écoulement de la vapeur dans l'ensemble de la figure 6
Figure 10 : Accessoire type II
Figure 11 : Accessoire de la figure 10 sans anse
Figure 12 : Accessoire de la figure 10 fixé à un purificateur-vapeur
Figure 13 : Accessoire de la figure 10 avec couvercle
Figure 14 : Accessoire de la figure 13 fixé à un purificateur-vapeur
Figure 15 : Écoulement de la vapeur dans l'ensemble de la figure 14
Figure 16 : Écoulement de la vapeur à travers le couvercle des figures 13 à 15.

Références numériques utilisées dans les figures :
1. Accessoire type I
2. Support
3. Sortie vapeur
4. Objet tubulaire
5. Objet cylindrique
6. Objet de forme quelconque
7. Entrée vapeur
8. Connecteur fluidique
9. Buse
10. Tube creux
11. Élément annulaire d'extension de support
12. Purificateur-Vapeur
13. Réservoir condensat
14.Accessoire type II
15. Récipient
16. Anse
17. Grille
18. Couvercle
19. Orifice évacuation vapeur
20. Fente évacuation vapeur

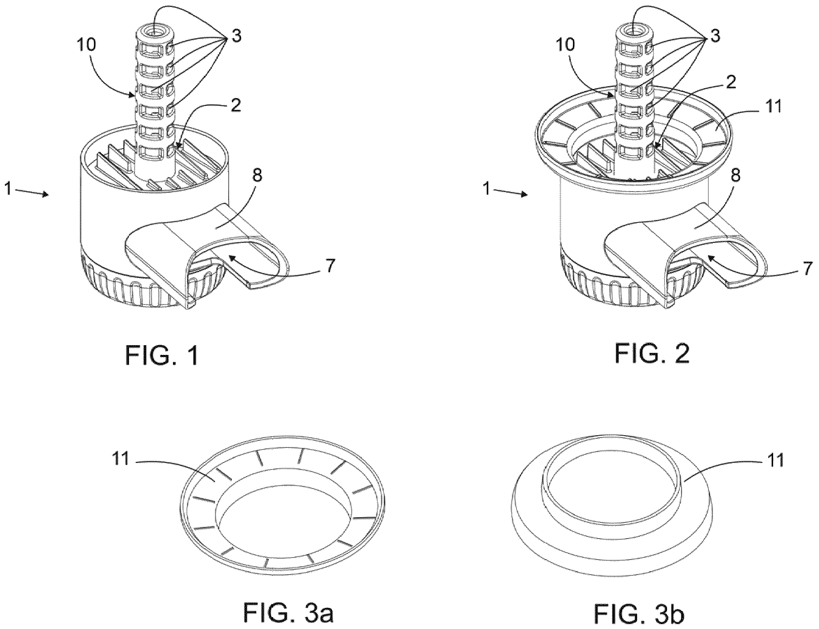

Les figures 1 à 9 représentent un premier type d'accessoire **1** selon l'invention destiné à la purification d'objets creux **4,5** dont la cavité doit être traitée au moyen de vapeur.

L'accessoire **1** comprend un support **2** formé d'une surface sur laquelle sont disposées plusieurs lames orientées verticalement. Au milieu du support **2** se trouve un tube creux **10** contenant plusieurs sorties de vapeur **3,** réparties sur sa face latérale, à l'exception d'une sortie **3** qui est disposée sur face supérieure. Le tube creux **10** est orienté perpendiculairement par rapport à la surface du support **2.**

Dans l'exemple illustré, le support **2** se présente sous la forme d'un disque. Selon d'autres variantes non-illustrées, le support a d'autres formes, p.ex. polygonale (carré, hexagone, etc...).

Tout type d'objet creux nécessitant un traitement de sa cavité interne peut être utilisé. A titre non-exhaustif, il peut s'agir de bouteilles, de gourdes, de biberons, de bocaux, de boîtes de rangement à géométrie quelconque (cubique, rectangulaire, tétraédrique, etc...) ou de tubes ouverts à leurs deux extrémités.

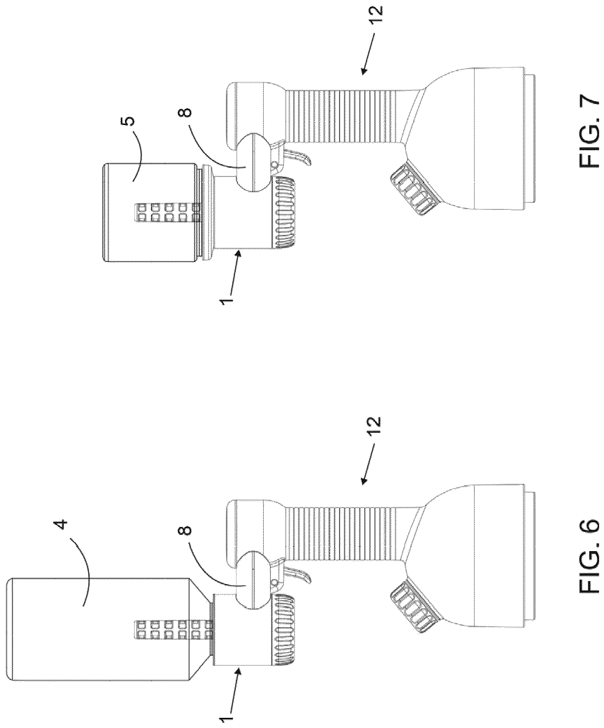

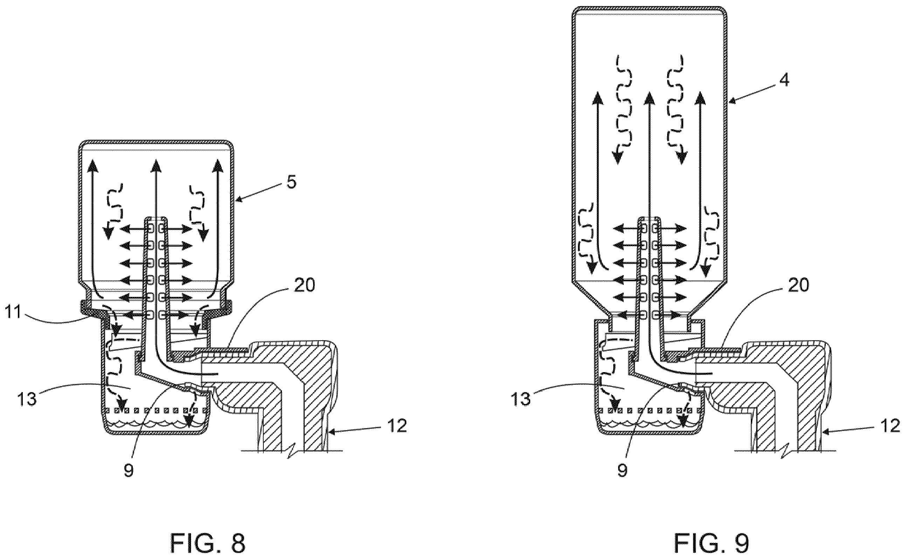

Les figures 6 et 9 représentent une configuration dans laquelle l'objet est une bouteille/gourde **4.**

Les figures **7** et 8 représentent une configuration dans laquelle l'objet est un bocal **5.**

L'objet **4,5** est disposé de manière à ce qu'au moins une de ses ouvertures fait face contact le support **2.**

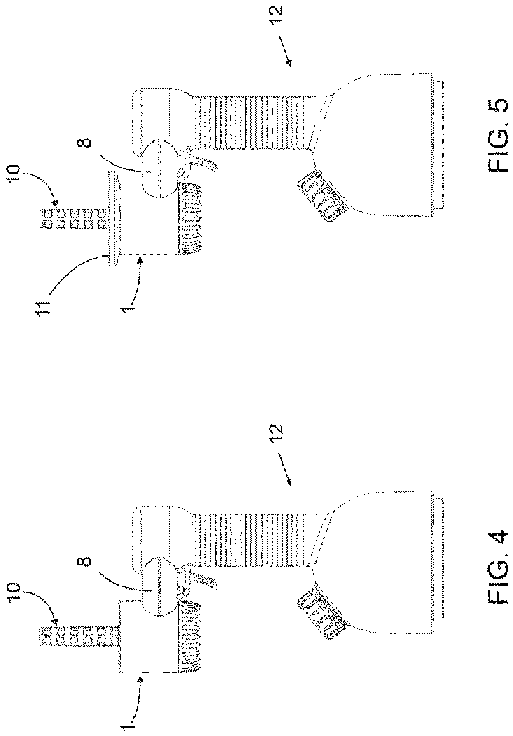

L'accessoire **1** comprend également un connecteur fluidique **8** fixé perpendiculairement à la paroi latérale du support **2.** Le connecteur **8** comprend une entrée de vapeur **7** qui communique avec les sorties de vapeur **3** du tube creux **10.** L'extrémité libre du connecteur **8** est dimensionnée de manière à assurer une fixation amovible étanche avec la buse **9** d'un purificateur-vapeur **12,** de préférence du type portable (figures 4 à 9).

Pour le traitement d'objets de diamètre relativement large **5,** la surface du support **2** peut être étendue au moyen d'un élément d'extension **11** de forme annulaire (figures 2, 3a et 3b).

Selon une variante non-illustrée de l'invention, le support comprend plusieurs tubes creux.

Lorsque le purificateur-vapeur **12** est activé, la vapeur émise de la buse **9,** traverse le connecteur fluidique **8** et pénètre dans la cavité de l'objet **4,5** via les sorties de vapeur **3** du corps creux **10.**

Si la cavité de l'objet **4, 5** ne présente qu'une ouverture, comme c'est le cas dans les exemples illustrés, la vapeur se condense et se dirige par gravitation vers le support **2** sous lequel est disposé un réservoir de récupération du condensat **13.** Celui-ci peut être vidé au moyen d'un couvercle (illustré sur les figures 8 et 9 par une ligne pointillée).

Une fente **20** d'évacuation de vapeur est disposée vers l'extrémité libre du connecteur fluidique **8** (figures 8 et 9).

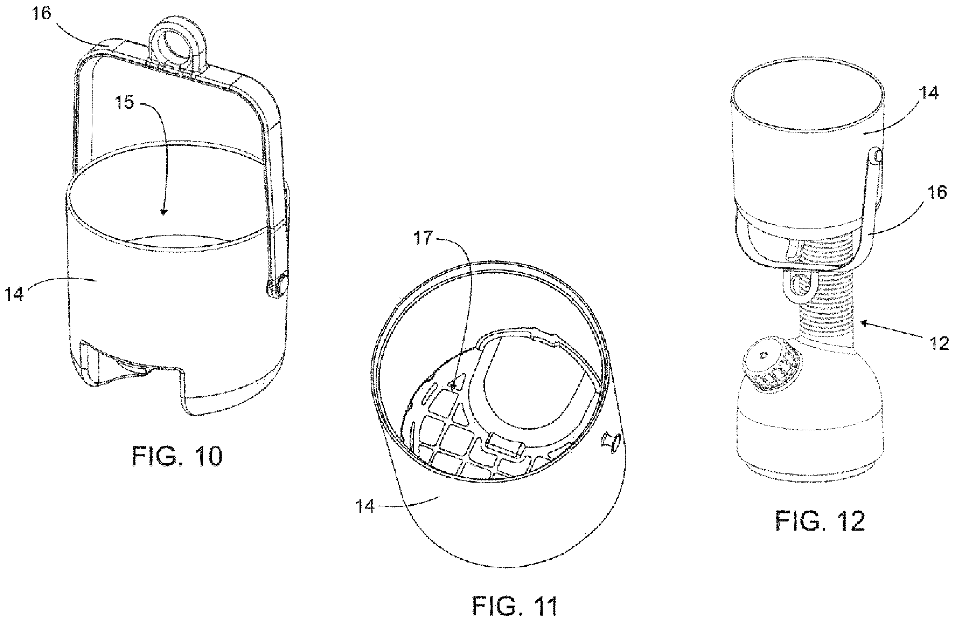

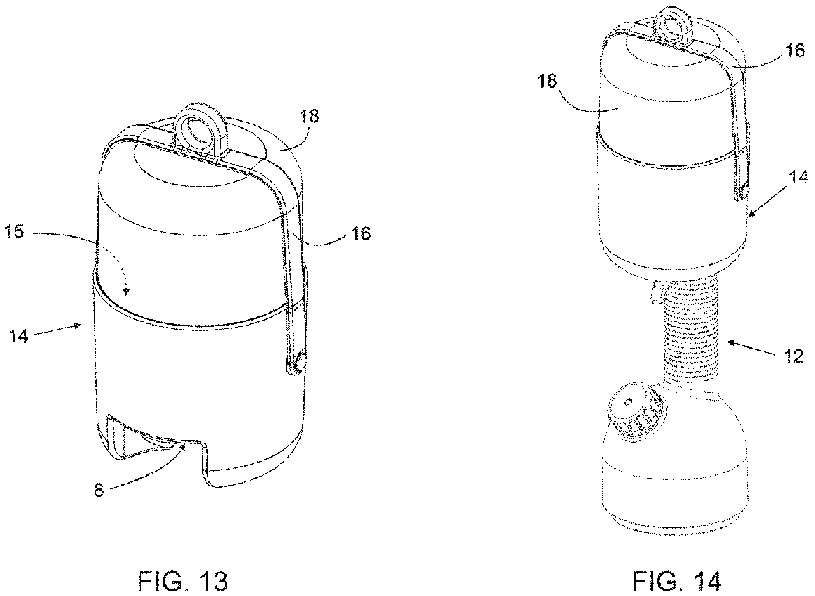

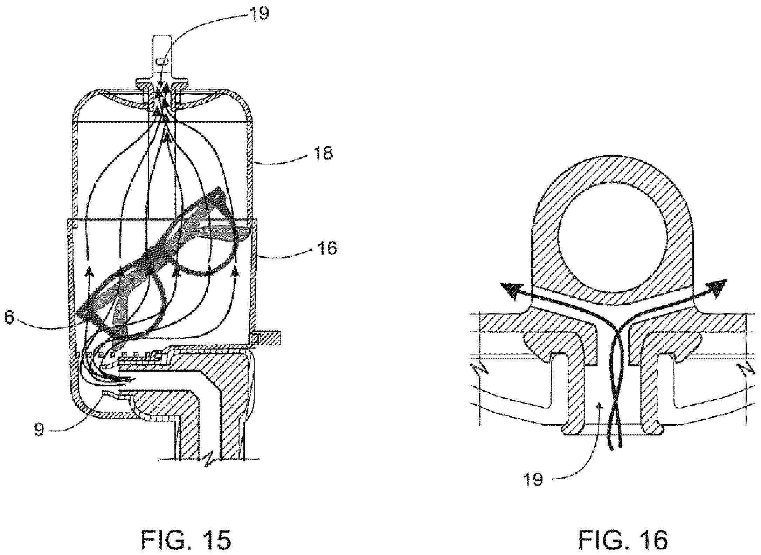

Les figures 10 à 16 représentent un deuxième type d'accessoire **14** selon l'invention destiné à la purification d'objets de dimensions relativement faibles et/ou diverses, comme p.ex. des lunettes **6** (figure 15), des clés ou des ustensiles.

Dans cet exemple, le support est un récipient **15** dont le fond est une grille **17** (figure 11) au travers de laquelle la vapeur circule.

De préférence, le récipient **15** est utilisé avec un couvercle **18.** La cavité ainsi formée améliore considérablement le traitement des objets **6.** Le couvercle **18** est solidement fixé au récipient **15** au moyen d'une anse **16** pivotable. De la sorte, une pression relativement importante peut être générée dans la cavité.

Afin d'éviter une surpression, le couvercle **18** comprend un orifice d'évacuation de vapeur **19** (figures 15 et 16). La présence de cet orifice **19** permet également d'induire un flux de vapeur au sein de la cavité, qui s'écoule entre la buse **9** et l'orifice d'évacuation de vapeur **19.**

La présente invention ne se limite pas aux exemples illustrés. Comme indiqué précédemment, l'accessoire n'a pas forcément une forme cylindrique. Il peut p.ex. avoir la forme d'un parallélépipède.

De même, l'accessoire selon l'invention peut être formé de n'importe quel matériau adapté à l'usage envisagé.

## Revendications

1. Accessoire (1,14) pour purificateur-vapeur (12) comprenant un support (2, 15) d'objet (4-6) à purifier, au moins une sortie de vapeur (3) disposée de manière à assurer une vaporisation de l'extérieur et/ou de l'intérieur dudit objet (4-6), une entrée de vapeur (7) communiquant avec ladite sortie (3) ; **caractérisé par le fait qu'**il comprend également un connecteur fluidique (8) permettant de relier ladite entrée (7) à une buse de purificateur-vapeur (9) et de fixer de manière amovible l'accessoire (1,14) à ladite buse (9).

2. Accessoire (1,14) selon la revendication 1 dans lequel la communication fluidique entre ladite entrée (7) et ladite sortie (3) s'écoule à travers le support (2).

3. Accessoire (1,14) selon la revendication 1 de forme cylindrique et dont le connecteur (8) est orienté perpendiculairement par rapport à l'axe de symétrie de l'accessoire (1,14).

4. Accessoire (1,14) selon l'une quelconque des revendications précédentes comprenant un réservoir de condensat (13).

5. Accessoire (1) selon l'une quelconque des revendications précédentes comprenant un tube creux (10) comportant plusieurs sorties de vapeur (3) et dont l'une des extrémités est fixée au support (2).

6. Accessoire (1) selon la revendication 5 comprenant un élément d'extension de support monté de manière amovible sur le support (2).

7. Accessoire (1) selon la revendication 6 dans lequel le support (2) forme un disque et ledit élément d'extension (11) un anneau qui se fixe et s'étend le long de la périphérie du disque.

8. Accessoire (14) selon l'une quelconque des revendications 1 à 4 dans lequel le support constitue le fond d'un récipient (15).

9. Accessoire (14) selon la revendication 8 dans lequel le récipient (15) comprend un couvercle (18).

10. Accessoire (14) selon la revendication 9 dans lequel le couvercle (18) comporte au moins un orifice (19) pour l'évacuation de vapeur vers l'extérieur de l'accessoire.
